# EUROPEAN PATENT APPLICATION

(11) **EP 4 751 636 A1**
(43) Date of publication of application: **03.06.2026**
(21) Application number: 25198368.0
(22) Date of filing: 27.08.2025
(51) Int. Cl.: A61B 1/00, A61B 1/002, A61B 1/307

(54) **HAND SURGICAL INSTRUMENT WITH PROXIMAL SEALING ELEMENT**

(30) Priority: 28.11.2024 DE 102024135158
(71) Applicant: Olympus Winter & Ibe GmbH, 22045 Hamburg (DE)
(72) Inventor: Schröder, Bastian, 22589 Hamburg (DE); Schulz, Kevin Alexander, 22889 Tangstedt (DE)
(74) Representative: Hoener, Matthias

(57) **Abstract**

The invention creates a hand surgical instrument with a seal to an optic that is easy and inexpensive to manufacture and can be thoroughly cleaned. This is achieved by a hand surgical instrument having a main body with a shaft. In this instrument, the main body has at least one channel-like bore for an optic. This bore can merge into a tube section in the proximal direction, so that a telescope including optics can be installed through the tube section and the bore in the distal direction of the instrument. A distal end of the main body can be coupled with the shaft for the optics and a proximal end with the telescope. A longitudinal axis of the instrument can be defined by the bore and the tube section. A key feature is that a sealing body with a concentric sealing lip is arranged axially to this longitudinal axis in the proximal direction, whereby this sealing lip is oriented in the proximal direction.

## Description

The invention relates to a surgical instrument according to claim 1.

For urological applications or treatments or operations in the abdomen of persons, hand-held surgical instruments, such as endoscopes, cystoscopes, resectoscopes or the like, are known to be used. The instruments are guided with a tube-like shaft first through an opening in the body of the person to be treated and placed in the position intended for the application. Depending on the application or instrument, a wide variety of working instruments are guided through the shaft, such as forceps, scissors, clamps, electrode holders or the like. In addition or as an alternative to the aforementioned instruments, an optical system can be guided through the shaft into the body to provide visual control of the treatment.

Such optics are guided with an eyepiece and/or a camera as telescopes from a proximal direction of the instrument through the same, so that a distal end of the optics is brought into the distal area of the shaft in order to view the area directly in front of the shaft. For this purpose, the optic is first guided through a bridge or a main body of the instrument. This main body can have a tube section that leads through the main body or extends into the main body as a bore. A distal end of this main body can be coupled to the shaft. The optics or a telescope containing the optics can be attached to the proximal end or the tube section. For this purpose, it is conceivable that an optics plate is associated with this proximal end of the main body or the tube section.

During the patient's treatment, the area or space to be treated in the patient's body is flooded with a fluid. To ensure that the pressure of the fluid inside the body remains stable, it is important that all openings of the instrument are appropriately sealed. On the one hand, the seals prevent fluid from escaping from inside the body. At the same time, however, they also prevent any contamination from the outside entering the body through the instrument. Furthermore, these seals must be designed in such a way that, for example, the working instruments, but also the optics for the treatment to be carried out, can be moved back and forth in the axial direction of the instrument.

Known solutions for sealing the rod-shaped optic against the tube section or the main body provide for a ring-like seal to be arranged around the optic, which is provided with a sealing lip that wraps around the circumference of the optic. There are known embodiments in which the sealing lip is reinforced by a metal ring. This known seal is located either in the main body or in the optics plate at the proximal end of the tube section. The use of such a ring-like seal is disadvantageous because, viewed in the axial direction, impurities can accumulate in front of and behind the sealing lip, which can only be removed with increased effort or not at all. This makes the cleaning and reprocessing of such instruments extremely cost-intensive. Due to the geometry of the known seals, it is almost impossible to see from the outside whether the cleaning meets the high requirements or not. A further disadvantage of the known ring-type seal is that the sealing lip is folded forwards and backwards when the optics are pulled back and forth within the tube section or the main body. This can lead to increased mechanical wear. To prevent this, the seal is reinforced with a metal ring. However, this design is difficult to manufacture and therefore very cost-intensive.

Based on this, the problem of the present invention is to create a hand surgical instrument in which a simple, inexpensive and, above all, thoroughly cleanable seal can be produced for an optic.

A hand surgical instrument for solving this problem has the features of claim 1. Accordingly, it is provided that a hand surgical instrument, which may, for example, be an endoscope, a cystoscope, a resectoscope or the like, has a main body with a shaft. In this instrument, the main body has at least one channel-like bore for an optical system. This bore can merge into a tube section in the proximal direction, so that a telescope including optics can be installed through the tube section and the bore in the distal direction of the instrument. A distal end of the main body can be coupled with the shaft for the optics and a proximal end with the telescope. A longitudinal axis of the instrument can be defined by the bore and the tube section. A key feature is that a sealing body with a concentric sealing lip is arranged axially to this longitudinal axis in the proximal direction, whereby this sealing lip is oriented in the proximal direction. The sealing body is used to create a medium-tight connection between the instrument or the main body and the optics or the telescope. Unlike the prior art, a sealing ring is not closed around the rod-shaped optics. Rather, in the present invention, the sealing body forms a sealing connection with a surface of the telescope, which is attached to the main body. As soon as the telescope including the optics is brought into contact with the instrument or the rod-shaped optics is pushed into the tube section and the bore, the concentric sealing lip is pressed or pressed against the surface of the telescope. This means that there is no longer any direct contact between the sealing body and the rod-shaped optic, which reduces the mechanical load on the sealing lip. In addition, the described shape of the sealing body means that every area around the sealing body can be easily reached and inspected, so that cleaning or reprocessing after treatment can also be carried out in a simple and cost-effective manner.

Preferably, it is provided that the sealing body is arranged in the proximal direction in front of or at an opening of the tube section for receiving the optics. In particular, it may be provided that the sealing body is integrated in an optics plate, the optics plate being connected or couplable to the proximal end of the tube section. The seal between the instrument and the optics is thus positioned exactly at the location where the known seals are also arranged. Accordingly, it is conceivable that existing systems can be converted simply by replacing the sealant with the seal body. It has been shown that sealing at this position is particularly simple and efficient. In addition, this area is particularly easy to clean as it has no dead spaces, undercuts or the like.

Preferably, the invention provides that the concentric sealing lip of the sealing body projects beyond the optics plate in the proximal direction or that the concentric sealing lip is flush with the optics plate or that the concentric sealing lip is offset in the distal direction relative to the optics plate. It may be provided that the sealing lip protrudes a few tenths of a millimeter or 1 to 2 mm in the proximal direction beyond the optical plate, so that when coupling with the telescope a force must be applied in order to bring the telescope into contact with the optical plate against the spring force of the elastic sealing lip. This application of force and the associated mechanical tension within the sealing body ensures an adequate seal between the aforementioned components during the entire treatment. At the end of the treatment or when decoupling the telescope from the optical plate, the mechanical pretension of the sealing body serves to guide the telescope out of the instrument. Due to the pretension, the seal body pushes the telescope out of the instrument after the locking mechanism is released. It is intended that the concentric sealing lip is reversible and returns to its original shape when deformed by the application of force. This means that the sealing body can be used many times without losing its sealing properties.

A further preferred embodiment example provides for the concentric sealing lip of the sealing body to be aligned towards the longitudinal axis, i.e. to converge towards the longitudinal axis. This shape of the sealing lip means that when the telescope is joined to the instrument, the inner diameter of the ring-like or concentric sealing lip is reduced and a spring force is built up as a result. The sealing effect is then created between the slightly inwardly pressed sealing lip and the telescope.

Similarly, another preferred embodiment example may provide for the concentric sealing lip to diverge outwards away from the longitudinal axis. In this embodiment example, an outer diameter of the sealing lip increases when the telescope is installed inside the instrument or when these two components are pressed together. The sealing effect is formed between an inner wall of the sealing lip and the surface of the telescope. In this design example, the preload or the resilient effect acts axially outwards. As soon as the connection between the instrument and the telescope is released again, the sealing lip relaxes in the opposite direction.

Furthermore, according to the invention, it can be provided that an inner diameter of the sealing body is larger than an inner diameter of the tube section and/or the optics. Accordingly, the seal is not made between the sealing body and the optics.

A particularly advantageous embodiment of the invention can provide for an inner wall of the sealing body to be conical in the distal direction to the opening of the tube section, whereby the diameter of the inner wall is reduced in the distal direction. This conical design facilitates the insertion of the optics into the tube section or into the instrument. The conical sealing body behaves like a ramp and guides the optic into the tube section even if the insertion is slightly offset.

Finally, it may preferably be provided that the sealing body is made of a plastic, an elastomer, fluorocarbon rubber (FKM) or ethylene propylene diene rubber (EPDM). These materials have proven to be particularly suitable, as they meet the requirements for materials used in the medical field, have a high mechanical resistance and can be produced at low cost.

A preferred embodiment of the invention is explained in more detail below with reference to the drawing. This shows:
- Fig. 1: a schematic representation of a hand surgical instrument,
- Fig. 2a: a schematic sectional view of a first embodiment of a sealing body without telescope,
- Fig. 2b: a schematic sectional view of a first embodiment of a sealing body with telescope,
- Fig. 3a: a schematic sectional view of a second embodiment of a sealing body without telescope, and
- Fig. 3b: a schematic sectional view of a second embodiment of a sealing body with telescope.

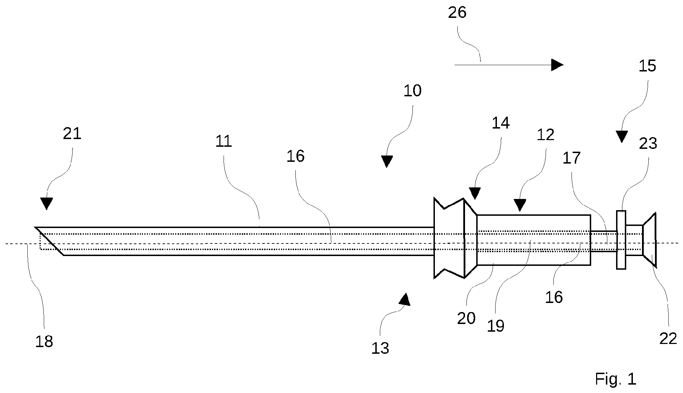

Fig. 1 shows a highly schematized hand-surgical instrument 10. This instrument 10 may, for example, be an endoscope, a cystoscope, a resectoscope or a similar instrument for minimally invasive treatment of a patient.

The hand surgical instrument 10 described here essentially consists of a tube-like shaft 11 and a bridge 12. To treat the patient, the instrument 10 is guided with a distal end 21 of the shaft 11 into an opening in the patient's body. The bridge 12 is located outside the body. Some embodiments of the instrument may provide for various working instruments to be introduced into the body through the shaft 11 via the bridge 12, such as wires, probes, clamps, catheters, stents, electrodes, flexible instruments or the like. In the case of the instrument shown in Fig. 1, for the sake of clarity, only an optic 16 is inserted into the shaft 11 via the bridge 12.

The shaft 11 can be coupled with a proximal end 13 to a distal end 14 of a main body 20 of the bridge 12. For example, a screw fastener, a click fastener, a bayonet fastener or a clamping ring can be used for this purpose. A proximal end 15 of the main body 20 has a tube section 17 to which an optical plate 23 is attached. A telescope 22 with the optics 16 can be coupled to this optics plate 23. An eyepiece or a camera can be attached to the telescope 22 so that the surgeon can view the area to be operated on via the rod lens system. As an alternative to the rod lens system, a fiber optic can also be used as an imaging device.

Within the main body 20, in the embodiment example of the hand surgical instrument 10 shown here, there is a bore 19. The bore 19 runs parallel to a longitudinal axis 18 and serves to accommodate the rod-shaped optics 16. The bore 19 extends out of the main body 20 into the tube section 17, which is an extension of the bore 19 along the longitudinal axis 18. This optic 16 can, for example, be a rod lens system not shown. This rod lens system extends from the proximal end 15 to the distal end 14 of the bridge 12 and is guided further through the entire shaft 11 to the distal end 21 of the shaft 11, where it is directed towards the area to be treated.

During the treatment, the interior of the body of the person to be treated is filled with a medium, preferably a liquid, in order to enlarge the interior to be treated via the pressure so that the surgeon has sufficient space for the treatment. All openings must be closed to prevent this medium or liquid from escaping from the body. This also includes the openings of the instruments used. A ring-like cavity extends between the shaft and the optics 16 from the distal end 21 to the proximal end 15. In order to close this cavity, the invention provides for a sealing body 24 to be arranged at the proximal end 15. This sealing body 24 has a concentric sealing lip 25 which is oriented in the proximal direction 26.

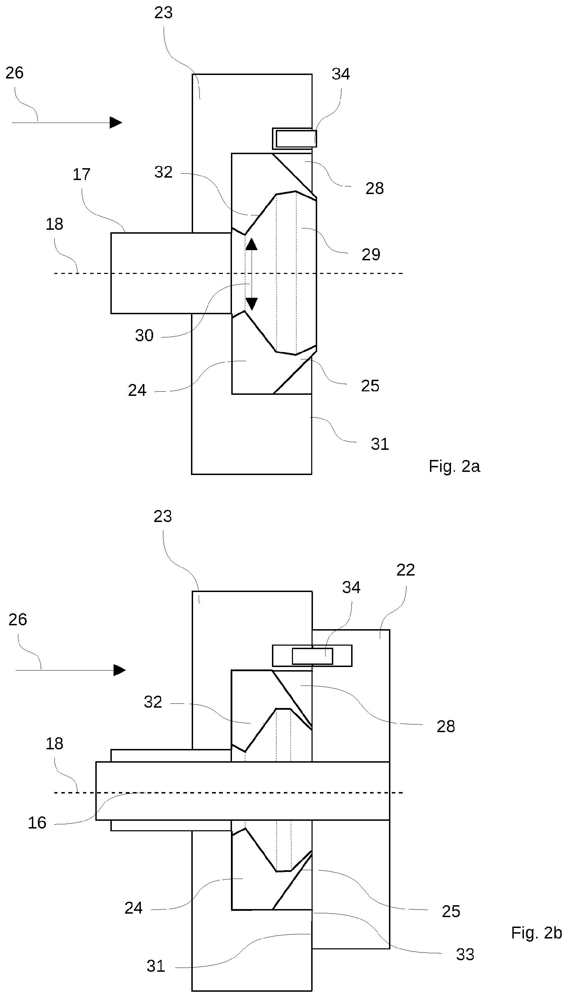

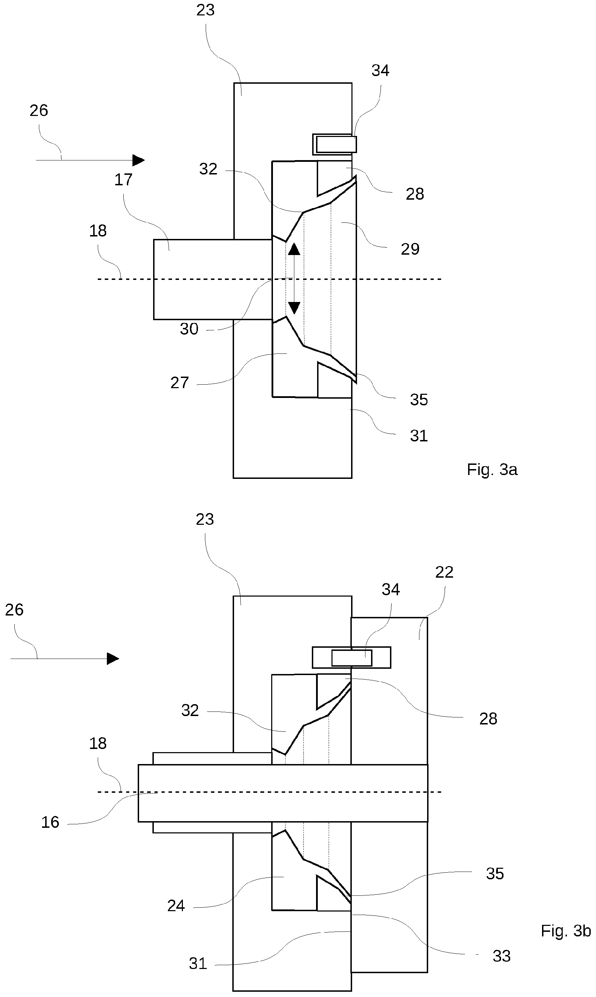

Figs. 2a, 2b, 3a and 3b show two possible embodiments of a sealing body 24, 27 according to the invention. For greater clarity, only part of the tube section 17 and the optical plate 23 are shown as a sectional view. The sealing body 24 according to the embodiment example shown in Fig. 2a is arranged in a receptacle 28 in the optical plate 23. This optical plate 23 is arranged on the pipe section 17, so that the sealing body 24 is located directly on or in front of the pipe section 17. The sealing body 24 is essentially ring-shaped and has a central opening 29. The optics 16 can be pushed through this central opening 29 into the tube section 17 against the proximal direction 26. For this purpose, the smallest inner diameter 30 of the sealing body 24 corresponds at least to the diameter of the pipe section 17.

In the proximal direction 26, the sealing body 24 has the concentric sealing lip 25, which extends wedge-like in cross-section in the proximal direction 26, namely beyond a contact surface 31 of the optical plate 23. This protrusion can be a few tenths of a millimeter up to 1 mm to 2 mm. However, embodiments are also conceivable in which the sealing lip 25 protrudes more than 2 mm beyond the contact surface 31. Due to the wedge-like design of the sealing lip 25, a wall thickness of the sealing body 24 or the sealing lip 25 tapers in the proximal direction 26. The sealing lip 25 according to this embodiment example points concentrically in the direction of the longitudinal axis 18, i.e. is oriented inwards.

An inner wall 32 of the sealing body 24 is conical in the direction of the tube section 17 and tapers from the opening 29 to the smallest inner diameter 30. This conical design of the inner wall 32 serves to ensure that the optics 16 can be inserted into the tube section 17 in a simple and reliable manner.

The sealing body 24 is made of an elastic material. Plastics and elastomers are particularly suitable, preferably fluororubber (FKM) or ethylene-propylene-diene rubber (EPDM). These irreversibly elastic materials have the advantage that they return to their original shape after deformation. If now - as indicated by Fig. 2b - the telescope 22 with the optics 16 is inserted into the bridge 12 or the shaft 11 and a sealing surface 33 of the telescope 22 is joined to the contact surface 31, the sealing body 24 or the concentric sealing lip 25 is deformed. In the process, the sealing body 24 or the concentric sealing lip 25 is deformed towards the longitudinal axis 18 by the sealing surface 33, so that the opening 29 of the sealing body 24 is reduced. During this deformation, a mechanical tension is built up within the sealing body 24, which results in the sealing lip 25 being pressed against the sealing surface 33. This pressing of the sealing lip 25 against the sealing surface 33 creates a sufficient seal in the ring-like space between the shaft 11 and the optics 16 so that no liquid can escape. To ensure that this tightness can be maintained during treatment, it is provided that the optical plate 23 is detachably coupled to the telescope 22 by a latching means 34.

Once the treatment is complete, the latching means 34 can be released and the contact surface 31 removed from the sealing surface 33. The surgeon is assisted by the pretension of the deformed sealing lip 25, which pushes the sealing surface 33 away in the proximal direction 26.

The use of this sealing body 24 is particularly advantageous, as on the one hand it creates the necessary tightness against leakage of the medium and on the other hand it is particularly easy and thorough to clean. It is conceivable that the sealing body 24 can be easily removed from the receptacle 28, for example to be replaced as required.

Fig. 3a schematically illustrates a further embodiment example of a sealing body 27. This sealing body 27 differs from the sealing body 24 only in the shape of the sealing lip 35. Since all other features shown are identical to the embodiment example shown in Fig. 2a, the same reference symbols are used and reference is made to the above description.

In contrast to the sealing lip 25, the sealing lip 35 is not oriented towards the longitudinal axis 18, but rather points concentrically outwards, whereby the opening 29 is enlarged. In this embodiment of the sealing body 27, when the sealing surface 33 of the telescope 22 is brought into contact with the contact surface 31 of the optical plate 23, the wedge-shaped sealing lip 35 is pressed outwards, i.e. concentrically away from the longitudinal axis 18. This state is shown schematically in Fig. 3b. Here too, a mechanical tension builds up within the sealing body 27 or the sealing lip 35, which presses the sealing lip 35 against the sealing surface 33. The mechanical tension is sufficient to create sufficient tightness between the bridge 12 and the telescope 22. Here too, after the treatment and after releasing the latching means 34, the sealing surface 33 is pushed in the proximal direction 26 by the mechanical force that is released.

This design example of the sealing body 27 proves to be just as advantageous as the previously described design example. This sealing body 27 also produces a high level of tightness and enables simple and thorough cleaning.

### List of Reference Numerals:

- 10: Hand surgical instrument
- 11: Shaft
- 12: Bridge
- 13: Proximal end
- 14: Distal end
- 15: Proximal end
- 16: Optics
- 17: Tube section
- 18: Longitudinal axis
- 19: Bore
- 20: Main body
- 21: Distal end
- 22: Telescope
- 23: Optical plate
- 24: Seal body
- 25: Sealing lip
- 26: Proximal direction
- 27: Seal body
- 28: Receptacle
- 29: Opening
- 30: Diameter
- 31: Contact surface
- 32: Internal wall
- 33: Sealing surface
- 34: Latching means
- 35: Sealing lip

## Claims

1. Hand surgical instrument (10), in particular an endoscope, a cystoscope or a resectoscope, having a main body (20) which has at least one channel-like bore (19) for optics (16), wherein the bore (19) merges in the proximal direction (26) into a tube section (17), and wherein a distal end (14) of the main body (20) can be coupled to a shaft (11) for the optics (16) and a proximal end (15) of the main body (20) can be coupled to a telescope (22), wherein a longitudinal axis (18) of the instrument (10) extends axially through the bore (19) and the tube section (17), **characterized in that** a sealing body (24, 27) is arranged axially to the longitudinal axis (18) in the proximal direction (26) with a concentric sealing lip (25, 35) which is oriented in the proximal direction (26).

2. Hand surgical instrument (10) according to claim 1, **characterized in that** the sealing body (24, 27) is arranged in the proximal direction (26) in front of or at an opening (29) of the tube section (17) for receiving the optics (16).

3. Hand surgical instrument (10) according to claim 1 or 2, **characterized in that** the sealing body (24, 27) is integrated into an optical plate (23), the optical plate (12) being connected or couplable to the proximal end of the tube section (17).

4. Hand surgical instrument (10) according to claim 3, **characterized in that** the concentric sealing lip (25, 35) of the sealing body (24, 27) projects beyond the optical plate (23) in the proximal direction (26) or that the concentric sealing lip (25, 35) is flush with the optical plate (23) or that the concentric sealing lip (25, 35) is offset in the distal direction (26) relative to the optical plate (23).

5. Hand surgical instrument (10) according to one of the preceding claims, **characterized in that** the concentric sealing lip (25, 35) is designed to be reversible and returns to its original shape when deformed by an application of force.

6. Hand surgical instrument (10) according to one of the preceding claims, **characterized in that** the concentric sealing lip (25) converges to the longitudinal axis (18).

7. Hand surgical instrument (10) according to one of the preceding claims, **characterized in that** the concentric sealing lip (35) diverges outwards away from the longitudinal axis (18).

8. Hand surgical instrument (10) according to one of the preceding claims, **characterized in that** an inner diameter (30) of the sealing body (24, 27) is larger than an inner diameter of the tube section (17) and/or the optics (16).

9. Hand surgical instrument (10) according to one of the preceding claims, **characterized in that** an inner wall (32) of the sealing body (24, 27) is conical in the distal direction towards the opening of the tube section (17), the diameter of the inner wall (32) being reduced in the distal direction.

10. Hand surgical instrument (10) according to one of the preceding claims, **characterized in that** the sealing body (24, 27) is formed from a plastic, an elastomer, fluororubber (FKM) or ethylene-propylene-diene rubber (EPDM).
